(19) Europäisches Patentamt
European Patent Office
Office européen des brevets

(11) Veröffentlichungsnummer: **0 490 175 B1**

(12) **EUROPÄISCHE PATENTSCHRIFT**

(45) Veröffentlichungstag der Patentschrift: **28.09.94**

(51) Int. Cl.5: **C07C 209/88**

(21) Anmeldenummer: **91120351.1**

(22) Anmeldetag: **28.11.91**

# (54) Verfahren zur Aufbereitung der Kristallisationsmutterlauge der Racematspaltung von 1-(4-Chlorphenyl)-ethylamin.

(30) Priorität: **11.12.90 DE 4039447**

(43) Veröffentlichungstag der Anmeldung:
**17.06.92 Patentblatt 92/25**

(45) Bekanntmachung des Hinweises auf die Patenterteilung:
**28.09.94 Patentblatt 94/39**

(84) Benannte Vertragsstaaten:
**BE CH DE ES FR GB IT LI NL**

(56) Entgegenhaltungen:
**EP-A- 0 341 475**

**Kirk-Othurer: Encyclopedia of Chemical Technology, 3rd Ed., Vol.7, pages 261-263**

(73) Patentinhaber: **BAYER AG**

**D-51368 Leverkusen (DE)**

(72) Erfinder: **Jansen, Johannes R., Dr.**
**Knipprather Strasse 47**
**W-4019 Monheim (DE)**
Erfinder: **Knops, Hans-Joachim, Dr.**
**Köpenickerstrasse 35**
**W-4019 Monheim (DE)**

**EP 0 490 175 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Aufbereitung der Kristallisationsmutterlauge, die bei der Racematspaltung von 1-(4-Chlorphenyl)-ethylamin der Formel (I) mit S-(-)-N-Phenylcarbamatmilchsäure der Formel (II) anfällt.

Diese Racematspaltung von 1-(4-Chlorphenyl)-ethylamin, z.B. zu R-(+)-1-(4-Chlorphenyl)-ethylamin, das u.a. als Zwischenprodukt für die Herstellung von fungizid wirksamen Verbindungen verwendet werden kann, beispielsweise durch Umsetzung mit S-(-)-N-Phenylcarbamatmilchsäure, ist bekannt (vgl. EP-A 341.475). Die bei diesem Verfahren anfallende ethanolische Kristallisationsmutterlauge enthält im wesentlichen S-(-)-1-(4-Chlorphenyl)-ethylamin und S-(-)-N- Phenylcarbamatmilchsäure bzw. deren Salz mit racemischem 1-(4-Chlorphenyl)-ethylamin. Bisher wurde diese Kristallisationsmutterlauge und somit teure Ausgangsverbindungen verworfen, da für die Isolierung dieser wertvollen Verbindungen aus der Mutterlauge kein technisch praktikables Verfahren bekannt war.

Aus Kirk Othmer "Encyclopedia of Chemical Technology" 3rd. Edition, Vol.7, 261 und 262 ist bekannt, daß ein in einem Solvens gelöster Stoff durch Zugabe eines weiteren als Fällungsmittel wirkenden Lösungsmittels zur Kristallisation gebracht werden kann. Es finden sich aber keine Hinweise darauf, daß ein bestimmter Stoff aus einem Gemisch auch dadurch isoliert werden kann, daß zunächst das vorhandene Lösungsmittel abdestilliert und dann ein anderes Solvens hinzugefügt wird.

Gegenstand der Erfindung ist nun ein Verfahren zur Aufbereitung der ethanolischen Kristallisationsmutterlauge, die bei der Racematspaltung von 1-(4-Chlorphenyl)-ethylamin der Formel (I) mit S-(-)-N-Phenylcarbamatmilchsäure der Formel (II)

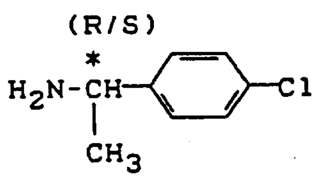

(I)

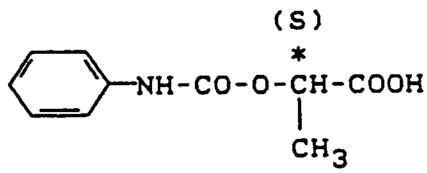

(II)

anfällt, welches dadurch gekennzeichnet ist, daß man in einem 1. Schritt das Lösungsmittel Ethanol bei einer Temperatur zwischen 0° C und 60° C unter vermindertem Druck abdestilliert und dann in einem 2. Schritt bei einer Temperatur zwischen 0° C und 60° C den verbleibenden Rückstand mit tert.-Butylmethylether oder Toluol verrührt und das kristallin anfallende Salz aus racemischem 1-(4-Chlorphenyl)-ethylamin und (S)-(-)-N-Phenylcarbamatmilchsäure der Formel

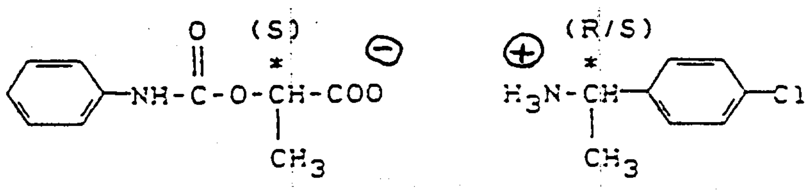

(III)

durch Absaugen isoliert.

Die tert.-Butylmethylether- oder Toluol-Mutterlauge enthält annähernd quantitativ das am (S)-Enantiomeren angereicherte 1-(4-Chlorphenyl)-ethylamin.

Es ist als ausgesprochen überraschend zu bezeichnen, daß mit Hilfe des erfindungsgemäßen Verfahrens auf einfache Weise die wertvollen Komponenten der Kristallisationsmutterlauge der oben beschriebenen Racematspaltung nahezu quantitativ zurückgewonnen werden können.

Dies gilt einesteils für die (S)-(-)-Phenylcarbamatmilchsäure, von der zur Racematspaltung von 1-(4-Chlorphenyl)-ethylamin insgesamt eingesetzten Menge (vgl. EP-A 341 475) ca. 80% verbraucht werden und als Salz mit (R)-1-(4-Chlorphenyl)-ethylamin anfallen; die restlichen ca. 20% der eingesetzten Menge werden beim erfindungsgemäßen Verfahren praktisch quantitativ zurückgewonnen, und zwar überraschenderweise als Salz mit racemischem 1-(4-Chlorphenyl)-ethylamin. Genau dieses Salz kann direkt wieder zur

Racematspaltung nach der bekannten Methodik (vgl. EP-A 341 475) eingesetzt werden. Die Isolierung der freien (S)-(-)-N-Phenylcarbamatmilchsäure aus dem Salz mit 1-(4-Chlorphenyl)-ethylamin ist nicht erforderlich. Damit ist der Teil von 1-(4-Chlorphenyl)-ethylamin, welcher racemisch als Salz mit (S)-(-)-N-Phenylcarbamatmilchsäure vorliegt, zurückgewonnen. In der tert.-Butylmethylether-Mutterlauge oder der Toluol-Mutterlauge liegt fast quantitativ das am (S)-Enantiomeren angereicherte Amin vor und kann auf übliche Weise der Weiterverwertung zugeführt werden, z.B. durch bekannte Racemisierungsverfahren und anschließende erneute Racematspaltung.

Ein besonderer Vorteil des erfindungsgemäßen Verfahrens liegt darin, daß dabei ganz auf die Verwendung von Wasser, welches sonst bei Aufarbeitungsprozefifien vielfach als Lösungsmittel verwendet wird, verzichtet werden kann. Damit werden kostspielige Destillationsschritte und Abwasserprobleme vermieden. Die beim erfindungsgemäßen Verfahren verwendeten organischen Lösungsmittel können hingegen mit wesentlich weniger Energieaufwand als Wasser destillativ zurückgewonnen und gegebenenfalls erneut in den Kreislauf eingeführt werden.

Das folgende Formelschema soll nochmals einen Überblick über den Ablauf geben; die angegebenen Mengenverhältnisse dienen nur der Illustration, d.h., sie sind angenäherte Werte, die aber nicht den präparativ angegebenen Werten voll entsprechen müssen:

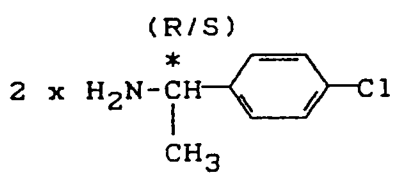
+
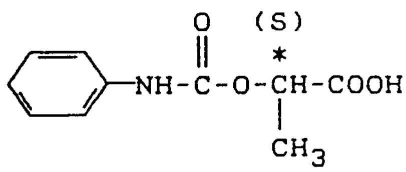

(I) (II)

Ethanol

**Kristallisat aus**
0,8 Mol (I) R-Form
mit
0,8 Mol (II) S-Form,

das zur Isolierung von reiner (I)-R-Form entsprechend EP-A 341.475 behandelt wird

**Kristallisationsmutterlauge aus:**
1,0 Mol (I) S-Form
0,2 Mol (I) R-Form
0,2 Mol (II) S-Form

erfindungsgemäß:

1. Ethanol-Abdestillieren
2. Zusatz von tert.-Butylmethylether oder Toluol

**Kristallisat aus:**
0,2 Mol (I) (R/S)-Form
mit
0,2 Mol (II) (S)-Form

**Mutterlauge aus:**
ca. 0,9 Mol (I) (S)-Form
0,1 Mol (I) (R)-Form

Die Reaktionsbedingungen für den 1. Schritt: Das Abdestillieren der flüchtigen Komponente (Ethanol) aus der Kristallisationsmutterlauge wird im allgemeinen bei Temperaturen zwischen 0 °C und 60 °C,

vorzugsweise zwischen 10 ° C und 50 ° C, insbesondere zwischen 20 ° C und 40 ° C, durchgeführt.

Das Abdestillieren der flüchtigen Komponente aus der Kristallisationsmutterlauge wird im allgemeinen unter vermindertem Druck, vorzugsweise bei Drücken zwischen 1 hPa und 100 hPa, insbesondere zwischen 5 hPa und 50 hPa, durchgeführt.

Der zweite Schritt, das Verrühren des Rückstandes mit tert.-Butylmethylether oder Toluol wird im allgemeinen bei Temperaturen zwischen 0 ° C und 60 ° C, vorzugsweise zwischen 10 ° C und 50 ° C, insbesondere zwischen 15 ° C und 45 ° C durchgeführt.

<u>Durchführungsbeispiele:</u>

<u>Beispiel 1</u>

Von 600 ml einer ethanolischen Kristallisationsmutterlauge, die bei der Racematspaltung von 1-(4-Chlorphenyl)-ethylamin der Formel (I) mit S-(-)-N-Phenylcarbamatmilchsäure der Formel (II) anfällt (gemäß EP-A 341 475), welche insgesamt 79 g (0,51 Mol) vorwiegend die (S)-Form von 1-(4-Chlorphenyl)-ethylamin und 18 g (0,086 Mol) (S)-(-)-N-Phenylcarbamatmilchsäure enthält, wird das Ethanol im Wasserstrahlvakuum bei 20 ° C bis 40 ° C abdestilliert. Der Rückstand wird mit 250 ml tert.-Butylmethylether mehrere Stunden bei 15 ° C bis 25 ° C gerührt, das kristalline Produkt durch Absaugen isoliert und mit 100 ml tert-Butylmethylether gewaschen. Man erhält 30,6 g (98% der Theorie) des Salzes von racemischem 1-(4-Chlorphenyl)-ethylamin mit (S)-(-)-N-Phenylcarbamatmilchsäure.

Von den vereinigten tert-Butylmethylether-Lösungen wird das Lösungsmittel im Wasserstrahlvakuum abdestilliert. Es verbleiben 60,4 g (ca. 0.38 Mol) an Roh-1-(4-Chlorphenyl)-ethylamin, wobei die (R)-Form zur (S)-Form ca. im Verhältnis 1:9 in diesem Rohprodukt vorliegt.

<u>Beispiel 2</u>

Von 600 ml einer ethanolischen Kristallisationsmutterlauge, die bei der Racematspaltung von 1-(4-Chlorphenyl)-ethylamin der Formel (I) mit S-(-)-N-Phenylcarbamatmilchsäure der Formel (II) anfällt (gemäß EP-A 341 475), welche insgesamt 79 g (0,51 Mol) vorwiegend die (S)-Form von 1-(4-Chlorphenyl)-ethylamin und 18 g (0,086 Mol) (S)-(-)-N-Phenylcarbamatmilchsäure enthält, wird das Ethanol im Wasserstrahlvakuum bei 20 ° C bis 40 ° C abdestilliert. Zum Rückstand werden bei einer Temperatur zwischen 30 ° C und 40 ° C 265 ml Toluol innerhalb von 30 Minuten gegeben und das Gemisch wird 12 Stunden bei 40 ° C gerührt. Dann wird das kristalline Produkt durch Absaugen isoliert (bei 20 ° C) und zweimal mit jeweils 50 ml Toluol gewaschen. Man erhält 31 g (99 % der Theorie) des Salzes von racemischem 1-(4-Chlorphenyl)-ethylamin mit (S)-(-)-N-Phenylcarbamatmilchsäure.

## Patentansprüche

**1.** Verfahren zur Aufbereitung der ethanolischen Kristallisationsmutterlauge, die bei der Racematspaltung von 1-(4-Chlorphenyl)-ethylamin der Formel (I) mit S-(-)-N-Phenylcarbamatmilchsäure der Formel (II) anfällt,

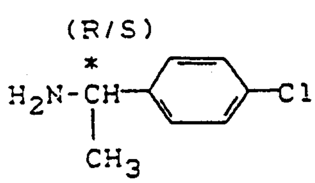

(I)

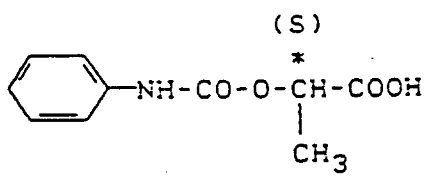

(II)

dadurch gekennzeichnet, daß man in einem 1. Schritt das Lösungsmittel Ethanol bei einer Temperatur zwischen 0° C und 60° C unter vermindertem Druck abdestilliert und dann in einem 2. Schritt bei einer Temperatur zwischen 0° C und 60° C den verbleibenden Rückstand mit tert.-Butylmethylether oder Toluol verrührt und das kristallin anfallende Salz aus racemischem 1-(4-Chlorphenyl)-ethylamin und (S)-(-)-N-Phenylcarbamatmilchsäure der Formel

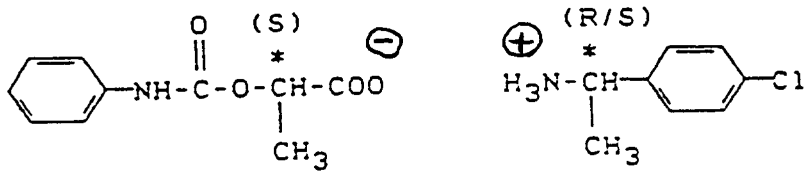

(III)

durch Absaugen isoliert.

2. Verfahren nach Anspruch 1, dadurch gekennzeichnet, daß der Druck zwischen 1 hPa und 100 hPa liegt.

## Claims

1. Process for working up the ethanolic crystallization mother liquor which is obtained in the resolution of the racemate of 1-(4-chlorophenyl)ethylamine of the formula (I) with S-(-)-N-phenylcarbamoyllactic acid of the formula (II)

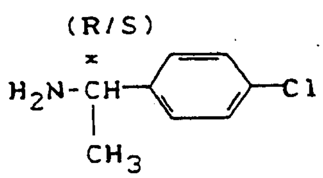

(I)

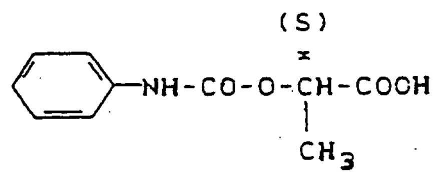

(II)

characterized in that in a 1st step the solvent ethanol is removed by distillation under reduced pressure at a temperature between 0 °C and 60 °C and then in a 2nd step the remaining residue is stirred at a temperature between 0 °C and 60 °C with tert.-butyl methyl ether or toluene and the resulting crystalline salt of racemic 1-(4-chlorophenyl)ethylamine and (S)-(-)-N-phenylcarbamoyllactic acid of the formula

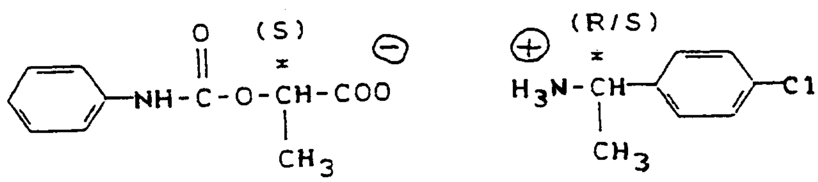

(III)

is isolated by filtering off with suction.

2. Process according to Claim 1, characterized in that the pressure is between 1 hPa and 100 hPa.

## Revendications

1. Procédé de traitement de la liqueur-mère éthanolique de cristallisation que l'on obtient dans la résolution du racémate de la 1-(4-chlorophényl)-éthylamine de formule (I) avec l'acide S-(-)-N-phénylcarbamate-lactique de formule (II)

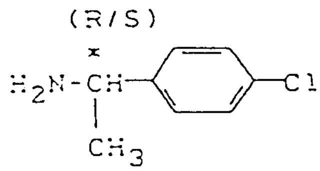

(I)

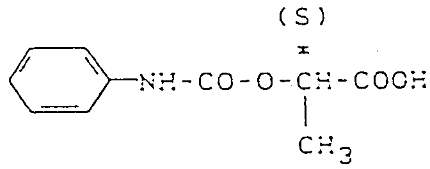

(II)

caractérisé en ce que, dans une première étape, on chasse le solvant éthanol par distillation à une température comprise entre 0 °C et 60 °C sous pression réduite puis, dans une seconde étape, on agite à une température comprise entre 0 °C et 60 °C le résidu restant avec de l'éther de tertio-butyle et de méthyle ou du toluène et on isole par filtration par succion le sel cristallin formé de la 1-(4-chlorophényl)-éthylamine racémique et de l'acide (S)-(-)-N-phénylcarbamatelactique de formule

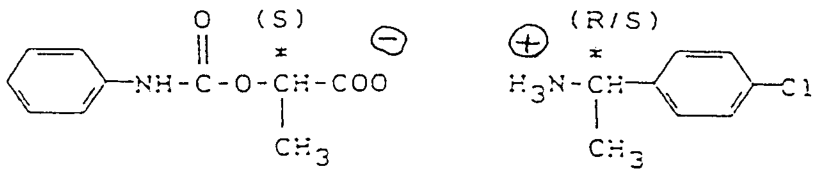

(III)

2. Procédé suivant la revendication 1, caractérisé en ce que la pression est comprise entre 1 hPa et 100 hPa.